(19) Europäisches Patentamt
European Patent Office
Office européen des brevets

(11) **EP 3 974 829 A1**

(12) **EUROPEAN PATENT APPLICATION**

(43) Date of publication:
**30.03.2022 Bulletin 2022/13**

(21) Application number: **20382847.0**

(22) Date of filing: **25.09.2020**

(51) International Patent Classification (IPC):
***G01N 33/531*** [(2006.01)]    ***G01N 33/543*** [(2006.01)]

(52) Cooperative Patent Classification (CPC):
**G01N 33/531; G01N 33/54346**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universidad de Castilla la Mancha 13071 Ciudad Real (ES)**

(72) Inventors:
• **VILLASEÑOR LLERENA, María Jesús**
  **13071 Ciudad Real (ES)**
• **RÍOS CASTRO, Ángel**
  **13071 Ciudad Real (ES)**
• **BARTOLOMÉ DÍAZ, Manuel**
  **13071 Ciudad Real (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **METHOD FOR ESTIMATION OF GLOBAL AMINO ACIDS CONTENT**

(57)    A novel, simple, quick and highly sensitive sensor based on citrate-functionalized gold nanoparticles (AuNPs) has been developed for both semi-quantitative and quantitative assessment of global free amino acids content.

EP 3 974 829 A1

**Description**

**FIELD OF THE INVENTION**

The present invention refers to the biochemical field. Particularly, it refers to a method for the assessment of global amino acids amount in a sample.

**STATE OF THE ART**

Amino acids are one of the most important organic molecules for the life existence as it is already known. These are characterized by having amino and carboxyl groups in their structure, responsible for their role as monomers in the building of proteins.

There have been developed a wide variety of methods for amino acids detection, mainly based on spectroscopic and separation techniques, e.g. mass spectrometry, fluorescence, high performance liquid chromatography (HPLC), capillary electrophoresis and gas chromatography (CG). In general terms, these methods usually require very expensive and complex instrumentation, qualified staff and high analysis times with laborious sample pre-treatment stages in numerous cases.

Latest trends for the development of sensors point to use metal nanoparticles (NPs), due to their unique physicochemical properties. Specifically, spherical AuNPs exhibit useful optoelectronic properties, reasons why they have become candidates for the construction of novel optical (colorimetric/spectrophotometric/fluorescent) sensor devices applied to detection of a wide range of analytes from very different nature, including amino acids, heavy metals and other organic molecules of interest.

However, in spite of the large amount of spectrophotometric methods for single amino acids detection, only very few ones allow quantification of their overall content while they are also able to maintain selectivity against other interferences. Thus, it would be desirable the development of easier and more intuitive methodologies with this same aim.

Thus, the present invention is focused on solving the above problem and it is herein proposed a method for the determination of the global amino acid content that may be present in a sample (not only specific amino acids). Apart from being able to determine the global amino acid content in a sample, the method of the invention is fast, simple, offers a high sensitivity and reduced pollution.

**DESCRIPTION OF THE INVENTION**

**Brief description of the invention**

The inventors of the present invention have designed a method for determining the total or global amount of amino acids which may be present in a sample. Specific nanoparticles and media conditions have been used in the method of the present invention in order to be able to detect the whole amino acid content (and not only specific amino acids) in a fast and simple way, while offering high sensitivity and reduced pollution.

Particularly, citrate-functionalized golden nanoparticles (AuNPs) have been used in the present invention. Spherical AuNPs have useful optoelectronic properties which make them good candidates for the construction of novel optical sensor devices applied for detection of a wide range of analytes, including amino acids. The optical properties of AuNPs derive from a physical phenomenon called Localized Surface Plasmon Resonance (LSPR), which is responsible of an intense absorption LSPR band exhibited by AuNPs in colloidal dispersions. The LSPR phenomenon causes the shift of the 518 nm absorption band ($A_{518}$) exhibited by disperse nanoparticles, which now appears at 650 nm ($A_{650}$) with intensity proportional to aggregated NPs concentration.

The inventors of the present invention have demonstrated that there is a correlation between amino acids concentration and the aggregated state of the AuNPs, and thus it is possible to estimate the global concentration of amino acids in a sample by using the band shift produced when the particles aggregate. This estimation can be performed quantitatively (for example by measuring the absorbance ratio ($A_{650}/A_{518}$) by UV-Vis spectrophotometry and calculating the concentration of amino acids using a lineal regression equation) and/or semi-quantitatively (visually comparing the intensity of developed colour versus a colour guide established from known standards).

Moreover, the inventors of the present invention have defined the optimal parameters for the development of the sensor, namely:

| Parameter | Optimal value | Possible range | Figure |
|---|---|---|---|
| *Size* (nm) | 18 | 13 - 25 | 1.4.a |

(continued)

| Parameter | Optimal value | Possible range | Figure |
|---|---|---|---|
| *Citrate Functionalization* | Citrate Functionalization | Citrate Functionalization | 2.2 |
| pH | 2.6 | 2.4 - 2.8 | 3.1 |
| [AuNPs] (nM) | 0.2 | 0.2 - 1.6 | 3.3 |
| *Time of incubation* (min) | 3 | 0 - 30 | 3.2 |
| *Ionic strength* ([NaCl] mM) | - | 0-50 | 3.4 |
| Linearity range ($\mu$M) | - | 0.48-2 | 6 |
| *Analytical signal* | $\dfrac{A_{650}}{A_{518}}$ | $\dfrac{A_{630}}{A_{518}} - \dfrac{A_{670}}{A_{518}}$ | 6 |

So, the first embodiment of the present invention refers to an *in vitro* method for the estimation of global amino acids and/or small peptides content present in a sample which comprises:

a) Contacting the sample with a solution of citrate-functionalized gold nanoparticles, wherein the concentration of the gold nanoparticles is between 0.2 and 1.6 nM, the pH of the solution is between 2.4 and 2.8 and the average diameter size of the nanoparticles is between 13 and 25 nm, and
b) Performing a quantitative estimation by measuring the absorbance ratio of the mixture between bands $A_{630-670}/A_{518}$, or a semi-quantitative estimation by comparing the intensity of developed colour versus a colour guide established from known standards.

The second embodiment of the present invention refers to the use of a solution of citrate-functionalized gold nanoparticles, wherein the concentration of the gold nanoparticles is between 0.2 and 1.6 nM, the pH of the solution is between 2.4 and 2.8, and the average diameter size of the nanoparticles is between 13 and 25 nm, for the estimation of global amino acids and/or small peptides content present in a sample.

In a preferred embodiment, the gold nanoparticles have an average diameter size of 18 nm.

In a preferred embodiment, the concentration of the gold nanoparticles is 0.2 nM.

In a preferred embodiment, the pH of the solution is 2.6.

In a preferred embodiment, the absorbance ratio of step b) is between $A_{650}/A_{518}$.

In a preferred embodiment, the time of contacting the sample with the gold nanoparticles of step a) is up to 30 minutes, preferably 3 minutes.

In a preferred embodiment, the sample is selected from a nutritional supplement, a biological sample obtained from a subject, food samples or beverage samples.

In a preferred embodiment, the sample is a blood sample.

For the purpose of the present invention the following terms are defined:

- By the term "comprising" it is meant including, but not limited to, whatever follows the word "comprising". Thus, use of the term "comprising" indicates that the listed elements are required or mandatory, but that other elements are optional and may or may not be present.
- By "consisting of' is meant including, and limited to, whatever follows the phrase "consisting of'. Thus, the phrase "consisting of' indicates that the listed elements are required or mandatory, and that no other elements may be present.
- By "global amino acids or small peptide content" is meant the total amount of amino acids or of small peptides, that is, the sum of all the amino acids or small peptides that are or may be present in the sample.
- By "small peptide" is meant molecules that consist of between 2 and 50 amino acids.

**Brief description of the figures**

**Figure 1. 1.1**) absorption spectra from 1:10 dilution of the five synthetized AuNPs fractions; **1.2**) SEM images for every AuNPs fraction: a) 1st, b) 2nd, c) 3rd, d) 4th and e) 5th; **1.3**) EDS spectrum for 1st AuNPs fraction; **1.4**) Intensity distribution histograms as diameter function for every AuNPs fraction: a) $1^{st}$, b) $2^{nd}$, c) $3^{rd}$, d) $4^{th}$ and e) $5^{th}$.

**Figure 2. 2.1)** UV-Vis spectra and AuNPs colour sensing solutions for: a) amino acids absence, b) low amino acids amount and c) high amino acids amount; **2.2)** AuNPs aggregation mechanism in presence of lysine.

**Figure 3.** Results from physic-chemical conditions study: **3.1)** pH influence on the $A_{650}/A_{518}$ ratio in absence and presence of lysine; **3.2)** Incubation time influence on the analytical signal for different pH values; **3.3)** AuNPs concentration influence on $A_{650}/A_{518}$ ratio; **3.4)** Variation of the selected analytical signal with the ionic strength.

**Figure 4.** Influence of potential interferences in different ratios on the analytical signal of a 1.5 $\mu$M global amino acids mixture.

**Figure 5**. Obtained analytical signal ($A_{650}/A_{518}$) for the six checked amino acids (3 $\mu$M), in the preliminary physic-chemical conditions (pH= 2.5, 0.43 nM and 30 min as incubation time).

**Figure 6**. AuNPs UV-Vis absorption spectra in presence of increasing global amino acids amounts (0, 0.48, 0.6, 0.8, 1.0, 1.3, 1.6 and 2 $\mu$M) prepared and recorded in the physic-chemical selected conditions.

**Figure 7. A)** Colour guide corresponding to the same levels of calibration range (0, 0.48, 0.6, 0.8, 1.0, 1.3, 1.6 and 2 $\mu$M) for amino acids amount treated in the selected physic-chemical sensing conditions. **B)** Developed colours for AuNPs solutions in presence of commercial samples: Mincartil, Collmar, Pectox Lisine, and BCCA weider (1.6 $\mu$M global amino acids concentration) and BCCA powder (1.0 $\mu$M global amino acids concentration).

## Detailed description of the invention

A detailed description of the invention is herein provided by means of the following examples, without the intention of limiting its scope of protection.

## Example 1. Materials and Methods

### Example 1.1 Reagents and solutions

Hydrogen tetrachloroaurate (III) trihydrate (HAuCl$_4$ · 3H$_2$O, >99.9%), sodium citrate dihydrate (C$_6$H$_5$Na$_3$O$_7$ · 2H$_2$O, >99%), sodium hydroxide (NaOH, >98%), NaCl (>99%), KCl (>99%), Cr(NO$_3$)$_3$· 9H$_2$O (>99%), L-lisine (Lys), L-cysteine (Cys), L-arginine (Arg), L-tyrosine (Tyr), L-methionine (Met), L-histidine (His) and pyridoxine (>98%) were obtained from Sigma Aldrich (St.Louis, MO). L-ascorbic acid (99.7%), riboflavin (>98%) and HCl (37%) were bought from Panreac (Spain, Barcelona). All the reagents were of analytical grade and used without purification.

Nutritional supplements (Mincartill, Collmar, BCCA powder, BCCA weider) and pharmaceutical formulation (Pletox Lisine) were acquired in specialised shops and local pharmacy.

It should be noted these nutritional supplements are mainly composed of amino acids in variable range and nature. In addition, it contains another excipient compounds in smaller proportion, like vitamins, metal ions and different additives, as follow described.

- Mincartill: amino acids (aspartic acid, threonine, serine, glutamic acid, proline, glycine, alanine, cysteine, valine, methionine, isoleucine, leucine, tyrosine, phenylalanine, lysine, histidine, arginine, tryptophan), vitamins (A, B$_1$, B$_2$, B$_5$, B$_6$, C, D), metal ions (Ca, Mg, Mn), food stabilizers (carboxymethilcelulose) and anticaking agents (silicon dioxide, fatty acid magnesium salts).
- Collmar: amino acids (tyrosine, hydroxylysine, histidine, serine, arginine, alanine, aspartic acid, glutamic acid, hydroxyproline, proline, glycine, valine, tryptophan, threonine, phenylalanine, methionine, lysine, leucine, isoleucine) as small peptides, vitamins (Biotine, vitamin C), natural food flavorings and different microencapsulated oils.
- BCCA weider: amino acids (leucine, isoleucine, valine), vitamins (B$_6$), food stabilizers (carboxymethilcelulose), metal ions (Ca, Mg), anticaking agents (silicon dioxide, fatty acid magnesium salts) and food flavorings.
- BCAA powder flavour red berries: amino acids (leucine, isoleucine, valine), vitamins (B$_6$, B$_9$ y C), sweeteners (sucralose and potassium acetosulfame) and natural colorants.
- Pectox Lisine: amino acids (lysine carbocysteinate), sweeteners (aspartame, mannitol) and natural food flavorings.

All involved solutions were freshly prepared using ultrapure water 18.2 M$\Omega$ cm from a Milli-Q system (Millipore, Bedford, MA, USA) and the appropriate amount of analytical grade reagents.

The synthesis procedure for AuNPs solutions was adapted from Bastús et al. [N. G. Bastús, J. Comenge, and V. Puntes, "Kinetically controlled seeded growth synthesis of citrate-stabilized gold nanoparticles of up to 200 nm: size focusing versus Ostwald ripening, " Langmuir, vol. 27, no. 17, pp. 11098-11105, 2011]. Based on this, five different AuNPs fractions of increasing sizes were synthesized using a nucleation and growth methodology upon Turkevich-Frens method.

### Example 1.2 Synthetic matrix preparation

In order to reproduce the complex composition of commercial real samples for a reliable amino acid detection, a synthetic matrix was prepared containing characteristic electrolytes ($Cr^{3+}$, $Na^+$, $Cl^-$, $K^+$) and vitamins (riboflavin, ascorbic acid, pyridoxine) usually also present in the analysed nutritional supplements, upon a former bibliographic search. With this aim, amino acids, vitamins and electrolytes amounts were previously evaluated on eight commercial supplements and average values for them were calculated to elaborate it. This synthetic matrix was employed to carry out the study about the analytical performance characteristics for our quantitative sensing methodology and also to prepare the standards of the colour guide employed in the semi-quantitative tests; in all these studies the ratio between the different components of the matrix and the global amino acids amount was kept always constant. **Table 1** shows the selected composition for this synthetic matrix in the analysis of a 1.2 $\mu$M global amount amino acids solution.

**Table 1**

| Components | Concentration (nM) |
|---|---|
| $Cr^{3+}$ | 0.09 |
| $Na^+$ | 8.16 |
| $K^+$ | 0.60 |
| $Cl^-$ | 6.35 |
| Ascorbic Acid | 22.07 |
| Pyridoxine | 0.53 |
| Riboflavin | 0.23 |

*Matrix composition for a 1.2 $\mu$M global amino acids concentration*

### Example 1.3 Real samples pre-treatment

Solid powder nutritional supplements (Mincartill, Collmar, BCCA powder, BCCA weider) and pharmaceutical formulation (Pletox Lisine) were previously homogenised. 1 g of each one was ground later until a fine powder was obtained. Then, appropriate amounts of each sample (9.5, 4.3, 8.2, 7.5 and 19 mg respectively) were taken to 250 mL of Milli-Q water and subsequently 1:100 diluted, for the preparation of theoretical 1.6 $\mu$M total amino acids solutions (according amino acids amounts declared by the manufacturer) by fivefold, except for BBCA powder, which was prepared in a theoretical 1 $\mu$M amount. All the aliquots were sonicated for 15 minutes before measuring to help its solubilisation.

For Kjeldahl analysis, 0.5 g of the ground samples were introduced into the Kjeldahl digestion chamber to begin the digestion process and the subsequent distillation and titration stages. The results so obtained, in terms of nitrogen percentage, were transformed to amino acids content using the corresponding factors for every kind of food samples [P. A. I. Reagents. "Determinación de nitrógeno por el método Kjeldahl." https://www.itwreagents.com/uploads/1018122/A173_ES.pdf].

### Example 1.4 Apparatus and Instrumentation

UV-Vis absorption spectra were taken by a SECOMAM UVI Light X2 spectrophotometer using a 1.0 cm quartz cell at room temperature. Scanning electron microscopy (SEM) images for AuNPs characterization (size, shape and dispersity) were acquired by ZEISS GeminiSEM 500. This one was coupled to Energy Dispersive X-Ray Spectroscopy (EDX) system, provided with an Oxford X-Max sensor (80 mm window) for performing elemental analysis studies. Dynamic light scattering (DLS) and Z potential measurements to obtain hydrodynamic diameter and Z potential of AuNPs were performed using a Zetasizer Nano Series DLS ZEN3500 equipment.

A Mettler AE 240 analytical balance, a Selecta ultrasound bath and a Crison pHmeter with a combined glass electrode were also used.

For Kjeldahl analysis, the Büchi Digestion Unit K-424 and Büchi Distillation Unit K-314 were required.

**Example 1.5. AuNPs characterization studies**

The physical characteristics of AuNPs strongly influence their LSPR band (physical basis of the analytical signal for this sensing methodology). Therefore, an exigent and rigorous characterization of the different synthetized AuNPs may be necessary before to choose one of them as the most suitable sensing component.

• *UV-Vis characterization*

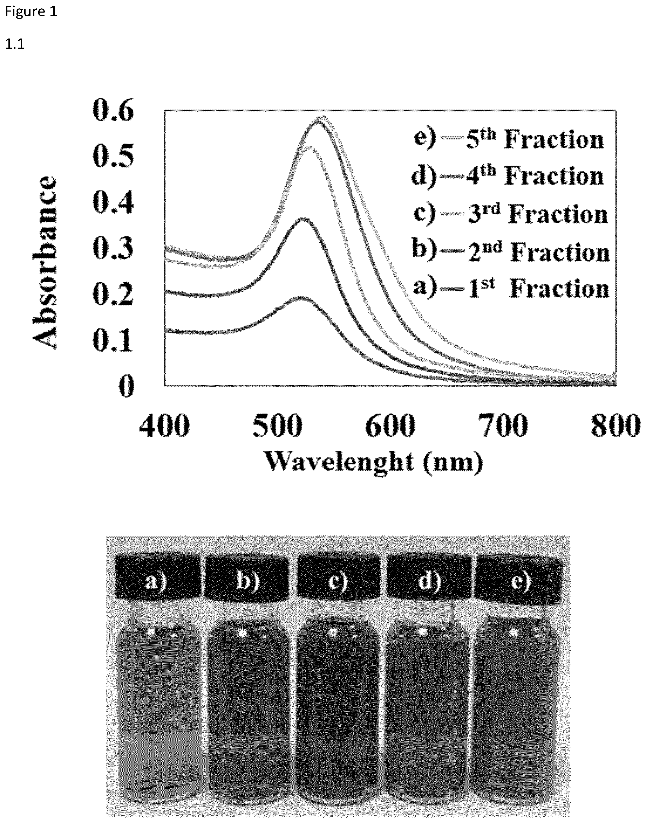

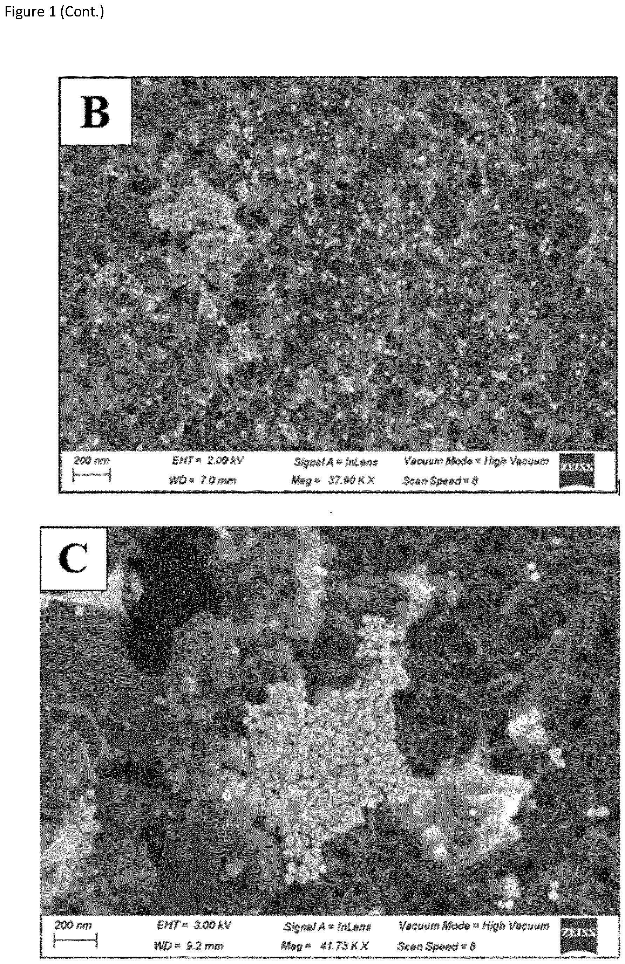

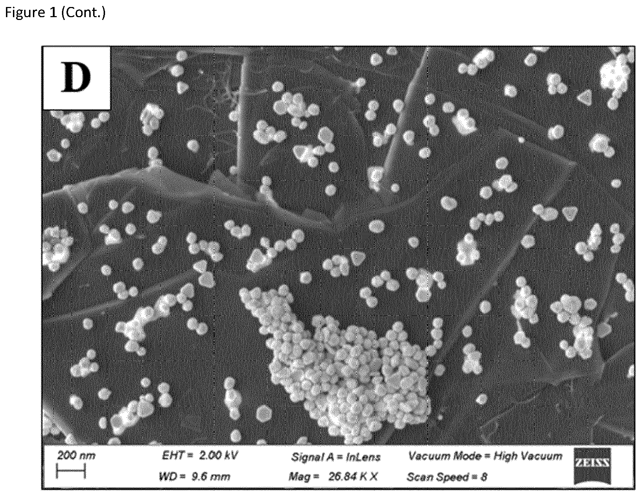

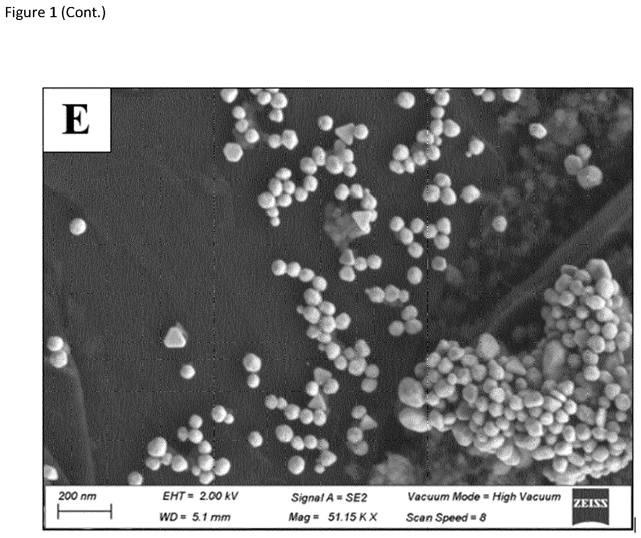

$\lambda_{SPR}$ was determined from UV-Visible absorbance measurements of 1:10 dilutions of the five different AuNPs synthetized solutions (**Figure 1.1**).
Particle size of those NPs having a diameter between 35 and 100 nm can be calculated from the position of the maximum in surface plasmon band using Equation 1:

$$d = \frac{\ln\left(\frac{\lambda_{SPR} - \lambda_0}{L_1}\right)}{L_2} \qquad (1)$$

where $\lambda_0$=512, $L_1$=6.53 and $L_2$=0.0216, are theoretical parameters from reference [31]. Size of smaller nanoparticles was determinate from $A_{SPR}/A_{450}$ rate according to Equation 2:

$$d = \exp\left(B_1 \frac{A_{SPR}}{A_{450}} - B_2\right) \qquad (2)$$

where $B_1$=3 y $B_2$=2.2 are experimental parameters from reference [31]. Smaller AuNPs fraction concentration were determined by using Equations 3 and 4 [32]:

$$C = \frac{N_T}{NV} \qquad (3)$$

where C is the AuNPs concentration (mol/L), V is the total solution volume (L), $N_T$ is the precursor amount (mol) and N is a factor related to nanoparticles size, "d" (nm)

$$N = 30{,}0896(d)^3 \qquad (4)$$

• *SEM characterization*

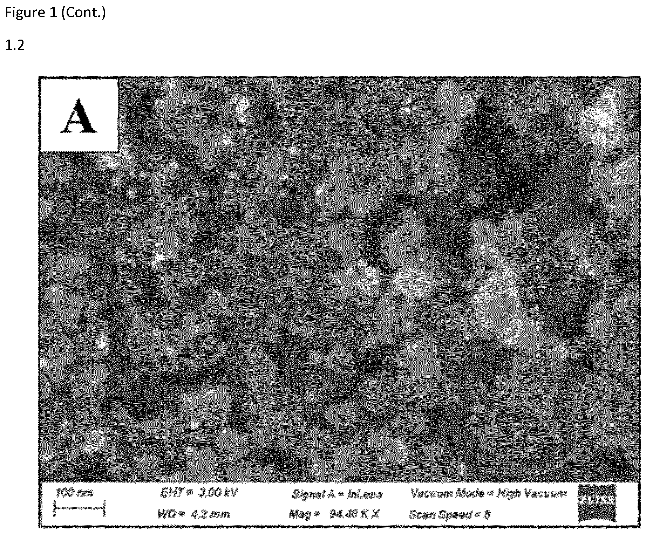

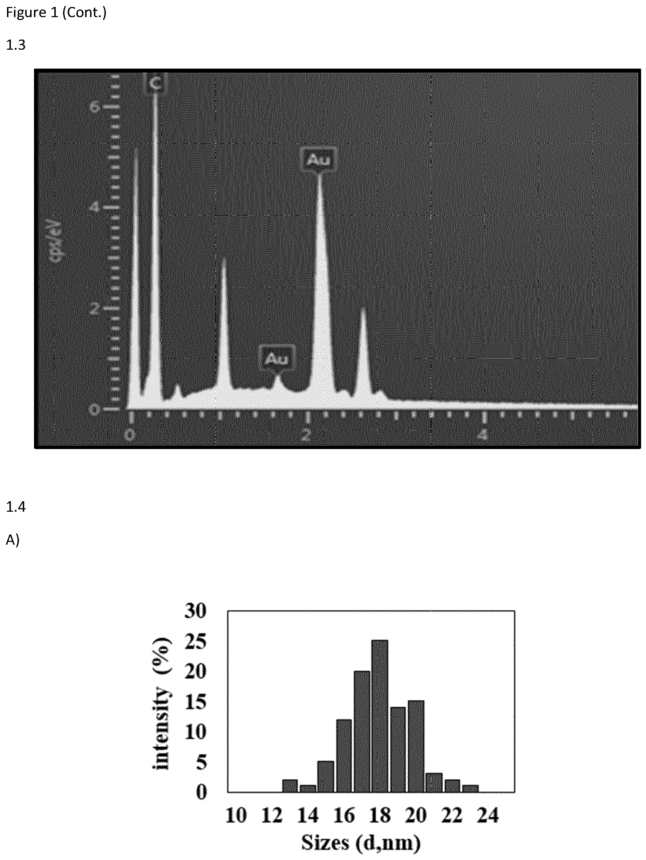

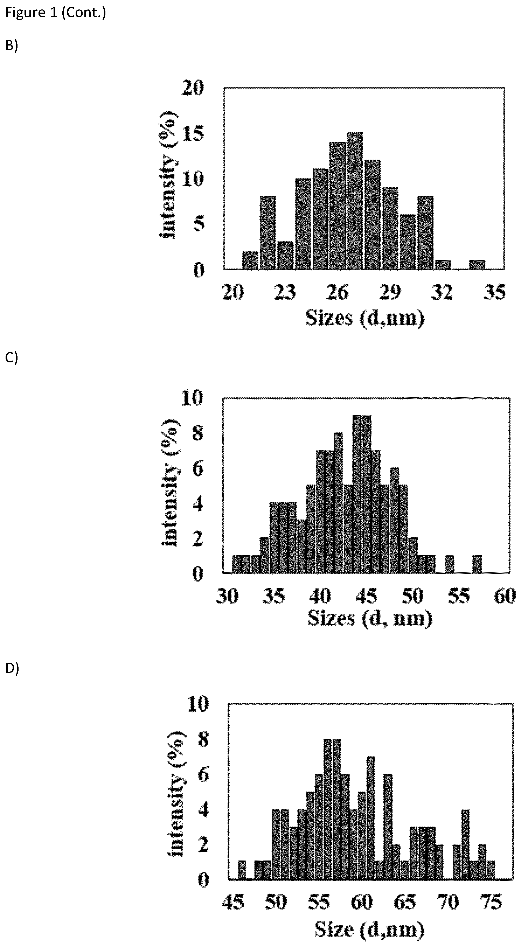

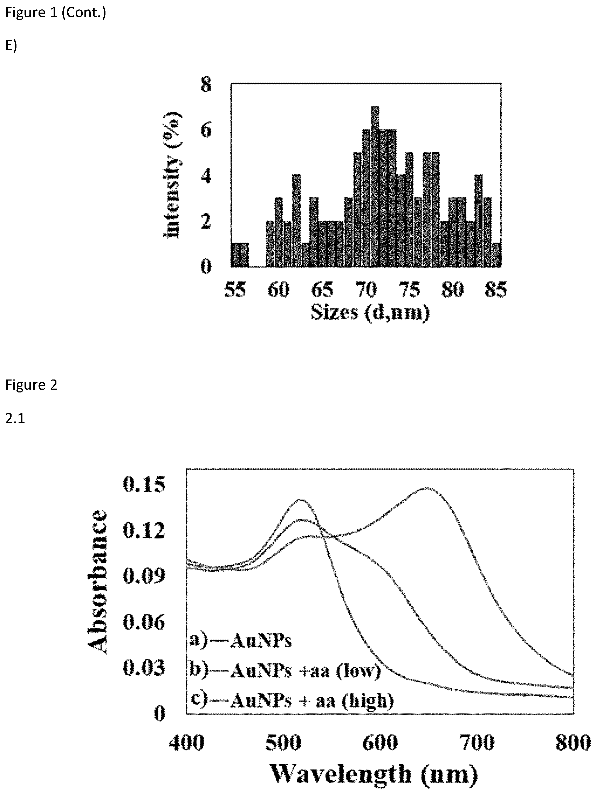

Size and morphology for AuNPs characterization were also checked by Scanning Electron Microscopy upon the procedure below described: 40 $\mu$L of each AuNPs fraction were deposited over different platforms (Si/SiO$_2$, Carbon and MWCNTs) and dried under infrared lamp. Only MWCNTs (multiwalled carbon nanotubes) platform allowed us to obtain clear images (**Figure 1.2**). EDS (energy dispersive X-ray spectroscopy) studies allowed us to check AuNPs elemental composition (**Figure 1.3**). The average SEM size and the intensity distribution histograms as diameter function for every AuNPs fraction (**Figure 1.4**), were obtained from the measure of 100 different nanoparticle's diameter.

• *DLS and Z Potential characterization*

Hydrodynamic diameters were obtained from Dynamic Light Scanning measurements by 1:5 dilution of the five different AuNPs synthetized solutions. This parameter was calculated as the medium value of 3 measurements. For Z potential determination, 1:5 diluted AuNPs solutions from every synthetized fraction were prepared and measured.
All the numerical results obtained in this exhaustive characterization study are shown in the **Table 2**.

**Table 2**

| Fraction | $\lambda_{SPR}$ UV-Vis (nm) | δ SEM size dispersity | UV-Vis size (nm) | SEM size (nm) | Hydrod. diameter (nm) | Z potential | Size reprod. RSD (%) |
|---|---|---|---|---|---|---|---|
| 1st | 518 | 11.1 | 17 | 18 ± 2 | 20 ± 4 | -39 | 3 |
| 2nd | 524 | 11.5 | 27 | 26 ± 3 | 28 ± 6 | -36 | 7 |
| 3rd | 528 | 14.0 | 41 | 43 ± 6 | 45 ± 9 | -35 | 9 |
| 4th | 534 | 13.3 | 56 | 60 ± 8 | 58 ± 14 | -31 | 11 |
| 5th | 541 | 13.3 | 69 | 73 ± 9 | 75 ± 16 | -27 | 11 |

*AuNPs Characterization results*

**Example 1.6. Colorimetric and spectrophometric UV-Visible procedure for amino acids detection**

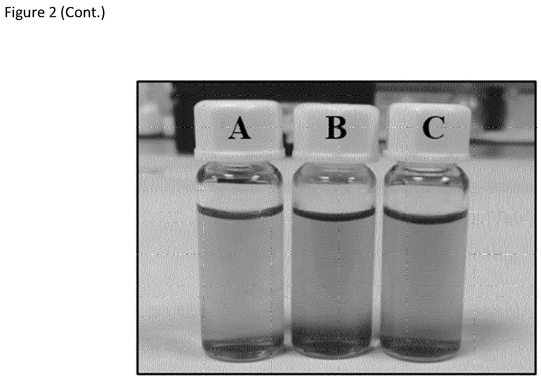

400 $\mu$L amino acids working solutions and 400 $\mu$L synthetic matrix solution were added to 2.90 mL HCl (3.5 mM) solution (final pH= 2.6), then 310 $\mu$L of the selected AuNPs solution were added to get 4 mL as final volume. After a period of 3 min as incubation time, the colour of solutions turned purple-blue, appearing a new band at 650 nm as well as decreasing the absorbance for that one at 518 nm, as consequence of the AuNPs aggregation (**Figure 2.1**). Then, UV-Vis spectra of solutions were registered in the range between 400 and 800 nm. The absorbance ratio between aggregation and dispersion bands, $A_{650}/A_{518}$, was selected as analytical signal related to amino acids amount for the quantitative sensing whereas the intensity of developed colour was compared versus a colour guide established from known standards as estimation of approximated amounts for the semi-quantitative tests.

**Example 2. Results**

**Example 2.1. AuNPs characterization**

Based on the obtained results (**Table 2**), it can be ascertained that the size of AuNPs measured by UV-Vis spectrophotometry (as well as their $\lambda_{SPR}$), SEM and DLS techniques, increased when advancing from the 1st to the 5th fraction. This fact corroborates the success of the selected procedure for the controlled AuNPs synthesis by means of a nucleation and growth methodology. It is also noticeable that very similar size estimations were obtained by means of the three techniques (SEM, DLS and UV-Vis spectroscopy). Moreover, the obtained SEM images (**Figure 1.2**) allow to verify that the synthesized AuNPs have pseudo-spherical geometry whereas the EDS results (**Figure 1.3**) confirm the nature of obtained NPs attending to the displayed peaks corresponding to carbon (SEM platform) and gold (AuNPs), thus evidencing their successful synthesis too.

**Table 2** also shows the size dispersity values obtained by SEM for every AuNPs fraction; size dispersity was calculated as p = ($\sigma$/ S) x 100, where "$\sigma$" was the standard deviation of AuNPs size and "S" is the average SEM size of AuNPs [I. Tiunov et al., "Synthesis of large uniform gold and core-shell gold-silver nanoparticles: Effect of temperature control," Russian Journal of Physical Chemistry A, vol. 90, no. 1, pp. 152-157, 2016]. These results report valuable data about the homogeneity of every fraction and they display lower polydispersity values for the first one as **Figure 1.4** also shows.

Regarding stability of the five different AuNPs sized synthetized, this was evaluated by means of Z potential measurements. Results in **Table 2** evidence how larger nanoparticle size involved more positive Z potentials, which mean less colloidal stability with higher aggregation tendency.

Finally, the reproducibility of the whole AuNPs synthesis procedure was evaluated for every fraction size (**Table 2**). With this aim, the global procedure was carried out in triplicate (n = 3) and the RSD values were calculated for every fraction from their UV-Vis sizes. Upon these results, lower reproducibility was obtained for longer diameter NPs, due likely to the higher number of involved steps.

As conclusion of these stability and reproducibility studies, the AuNPs with a size range between 13 and 25 nm where selected as transductor element for our sensing methodology because of its higher stability in solution (Z potential), lower polydispersity (p), smaller RSD size inter synthesis and lower synthesis time too. According to Equations 3 and 4, the concentration for this first AuNPs fraction was 2.59 nM.

**Example 2.2. Aggregation mechanism responsible of sensing system**

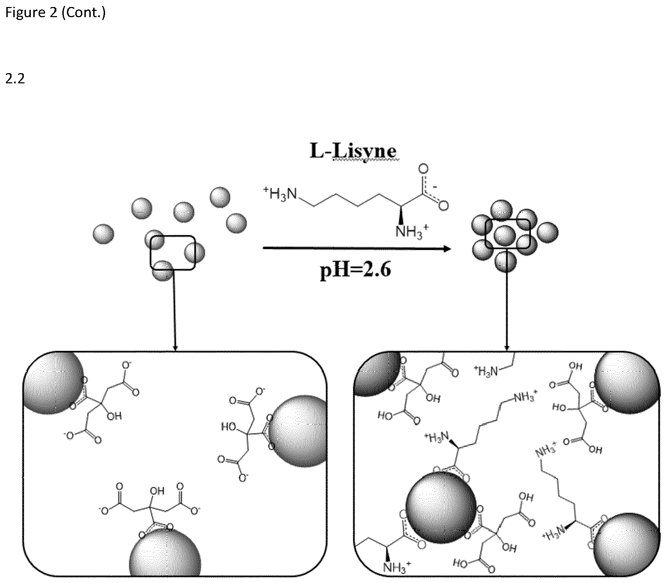

**Figure 2.2** illustrates the proposed aggregation mechanism using lysine as model amino acid.

According to pKas of both citric acid (pKa$_1$ = 3.15, pKa$_2$ = 4.77 and pKa$_3$ = 6.40) and lysine (pKa$_1$ = 2.16, pKa$_2$ = 9.06 and pKa$_3$ = 10.54) and as it can be appreciated on that figure, the aggregation takes place, on one side due to the interaction by hydrogen bindings between the protonated lysine amine groups and the oxygen non-binding electron pairs of the carboxylic citrate groups and, on the other side, due to the lysine carboxylate group, which form a coordination bond with the naked AuNPs surface; such phenomena would then give rise to the cross-linking mechanism responsible for aggregation.

Considering the functional groups involved in this aggregation, the exposed mechanism for lysine would be acceptable for any amino acid. In addition, other functional groups like thiols or any other additional amino or carboxylic ones may contribute to this aggregation mechanism, by forming new interactions with AuNPs too.

**Example 2.3. Selection of optimal physic-chemical conditions for the sensing system**

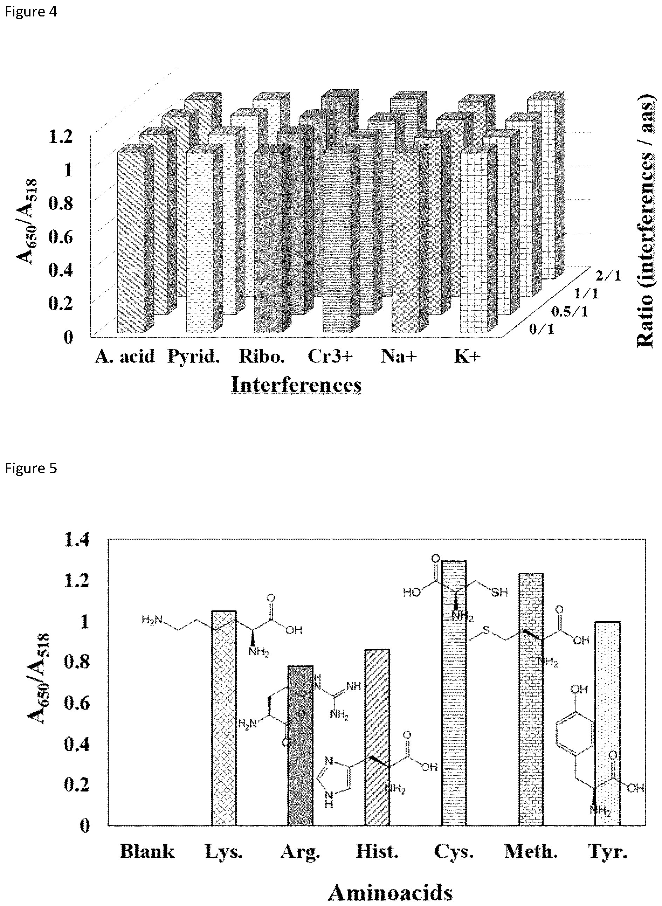

The selection of the suitable physic-chemical operating conditions was carried out taking lysine as representative analyte model, since it exhibited just the characteristic functional groups (amine and carboxylic ones) of amino acids, which assigned it a representative behaviour within them. However, the performance of the sensor was verified and evaluated for six different amino acids (**Figure 5**), chosen as representative ones for the main four categories within the so-called "20 natural amino acids". This classifying is based on a side chain nature criterion as follows: aliphatic (lysine, arginine), aromatic (tyrosine), with heterocycles (histidine) and with S (cysteine, methionine). All of them are also considered as essential amino acids, except cysteine and tyrosine, which are derived from their corresponding essential ones: methionine and phenyl-alanine respectively.

Physic-chemical conditions in terms of pH, incubation time, AuNPs concentration and ionic strength were optimized to obtain the best performance for the detection system. All the experiments were developed using a 3 $\mu$M lysine solution.

The effect of pH on the detection system was evaluated in a wide range (1.8-13) to look for the highest A$_{650}$/A$_{518}$ rate, which involved maximal sensitivity for our sensing methodology. These experiments were performed with 0.43 nM AuNPs and 30 min as incubation time as preliminary values for the six tested amino acids (**Figure 5**). To evidence that AuNPs aggregation specifically occurred by effect of the analyte the same tests were performed in absence of lysine too.

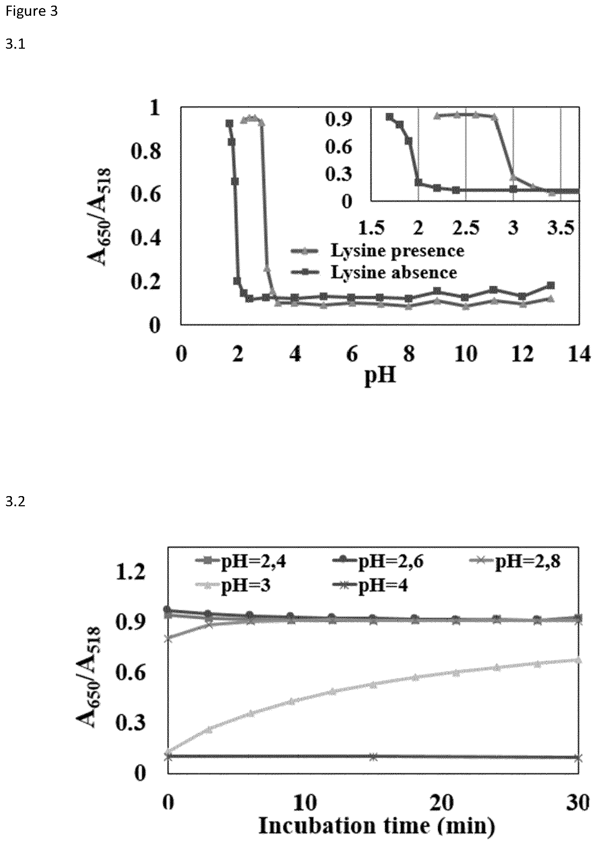

As it can be seen on **Figure 3.1**, in absence of lysine, our selected analytical signal (A$_{650}$/A$_{518}$) remain low and constant from 2.4 to 13 in the pH range, that means stable dispersed colloid, but for pH values $\leq$ 2.3, the colloid began to be disrupted with maximal aggregation for pH = 1.8, indicating poor AuNPs stability in extremely acidic medium.

In presence of lysine, AuNPs still remain dispersed in a pH range from 3.4 - 13, but for pH $\leq$ 3.4, lysine induces AuNPs aggregation, which is maximal in the range between 2.4 and 2.8. From these results, pH = 2.6 was selected as optimal one, since this value was far enough from the lower pH limit to avoid false positives and far enough too from the upper limit to guarantee maximal analytical signal.

To evaluate the influence of incubation time on the analytical signal, experiments were carried out at different pHs, recording UV-Vis spectra every 3 min. The obtained results are shown in **Figure 3.2**, where it can be aware for the suitable working pH range (2.4-2.8) and specially at the selected value, 2.6, maximal aggregation is immediately achieved by adding lysine; anyway, an incubation time of three 3 minutes was selected as safety measure.

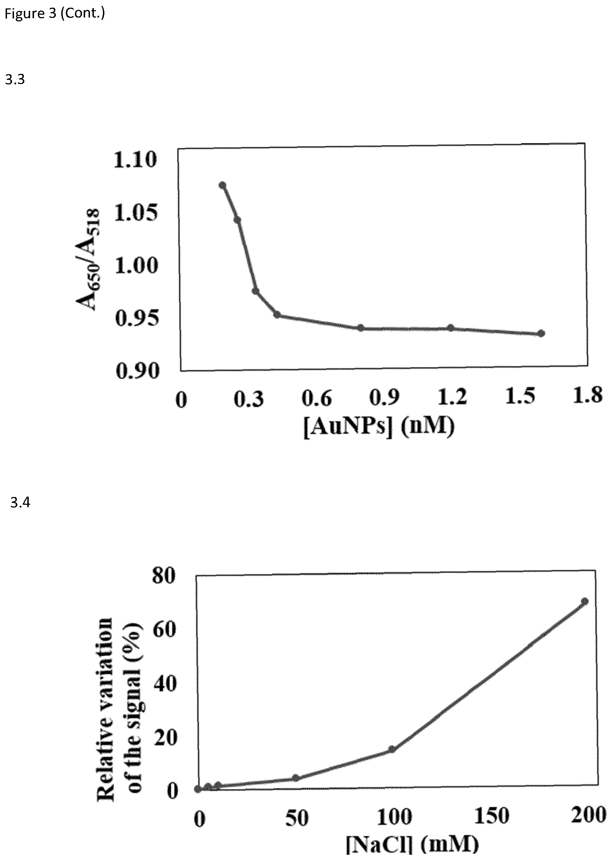

AuNPs concentration was evaluated too. With this purpose, different AuNPs concentration solutions in the 0.2 - 1.6 nM range were used for lysine 3 $\mu$M detection. As it is shown in the **Figure 3.3**, lower AuNPs concentration values lead to a A$_{650}$/A$_{518}$ rise, but AuNPs concentration values below 0.2 nM imply a very low absorbance/noise ratio, so it was decided to take 0.2 nM as the optimal concentration for the development of sensor.

Finally, to check the influence of the ionic strength on the selected analytical signal (A$_{650}$/A$_{518}$), the relative variation of this signal for a 3 $\mu$M lysine solution was evaluated by addition of NaCl in different concentrations (0-200 mM). In accordance with results displayed in **Figure 3.4**, it can be verified that the analytical signal variation let fall 5% for NaCl concentrations lower than 50 mM (that means more than 1.5 x10$^5$ times fold the analyte amount), which allowed us to perform measurements without appreciable errors (less than 5%).

**Example 2.4. Analytical Performance Characteristics for the global amino acid quantitative estimation**

These parameters were evaluated on the analytical signal obtained for synthetic matrix-analyte solutions consisting of a representative global amino acids mixture (the six ones previously evaluated in **Example 2.3**) and the synthetic matrix already mentioned (**Example 1.2**). The design of these synthetic matrix-analytes solutions attempted to reproduce the complex composition of commercial supplements for a reliable analytical features evaluation, where all solutions were treated in the selected physic-chemical conditions. This survey was performed upon internationally recognized EURACHEM guidelines [V. Barwick et al., "The fitness for purpose of analytical methods," 2014. https://www.eurachem.org/index.php/publications/guides/mv].

Precision of the proposed sensor was firstly checked using matrix-analytes solutions corresponding to 1.2 $\mu$M global amino acids mixture (composition in **Table 1**). For repeatability studies an RSD = 1.15% (one serie, n=7) was obtained. Sensor reproducibility was carried out at two exigency levels, obtaining RSD = 2.07% (two series with n = 7, t = 1-day, new prepared solutions except AuNPs) and RSD = 2.10% (two series with n=7, t=2 days, new prepared solutions including AuNPs too). Based on these results, it can be ascertained the good precision exhibited by the proposed sensing methodology; particularly noticeable results the fact that resynthesizing AuNPs solution had not effect over sensor precision.

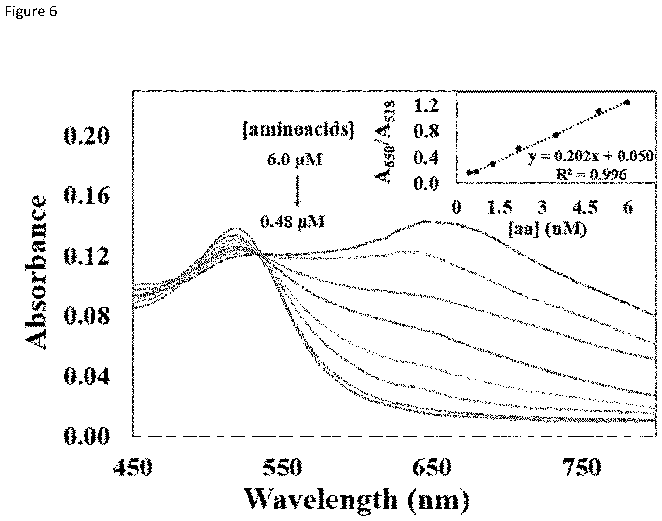

Linear response range was evaluated in triplicate by means of matrix-analyte solutions (n=7) with increasing global amino acids concentrations, prepared in the selected physic-chemical conditions and then recorded. Colour of these solutions was changing from red to purple, finally reaching an intense blue colour, as the amino acids concentration increased, which indicated AuNPs aggregation was gradually increasing due to the effect of total amino acids amount. **Figure 6** shows the UV-Vis spectra corresponding to this study, where it can be appreciated when amino acids concentration increases the intensity of the band located at 518 nm gradually decreases, as well as the band located at 650 nm becomes more intense. This behaviour causes an increase in the $A_{650}/A_{518}$ ratio (analytical signal), which maintains a good linear fit with global amino acids amount in the range between 480 nM and 2.0 $\mu$M and whose regression equation is: $Y(A_{650}/A_{518}) = 0.7254 X - 0.24$, with a determination coefficient of $R^2 = 0.996$.

LOD and LOQ values were 160 and 480 nM respectively. LOQ was empirically established as the lowest amino acids concentration able to be quantified and it was assigned to the first point of calibration range. According to literature LOD was defined as LOQ/3.33 [T. Wenzl, J. Haedrich, A. Schaechtele, P. Robouch, and J. Stroka, "Guidance Document on the Estimation of LOD and LOQ for Measurements in the Field of Contaminants in Feed and Food, " Publications Office of the European Union: Luxemburg, 2016].

Selectivity of this sensing mechanism for amino acids detection was assessed by means of an influence study about some potential interferences usually contained in nutritional commercial supplements (upon consulted literature), such as some stabilizers and anticaking agents, i.e. carboxymethilcelulose, silicon dioxide and fatty acids magnesium salts, which were removed by filtration of the samples and also with some vitamins and ions (ascorbic acid, riboflavin, pyridoxine, $Na^+$, $K^+$, $Cr^{3+}$) whose potential influence was checked. In this specificity study, solutions with different interference/global amino acids ratios (0:1, 0.5:1, 1:1 and 2:1) were prepared under the previously selected conditions about pH, [AuNPs] and incubation time and evaluated on the analytical signal ($A_{650}/A_{518}$) of 1.5 $\mu$M global amino acids mixture. Their influence was showed on **Figure 4**, where it can be estimated that our selected analytical signal ($A_{650}/A_{518}$) stay almost unchanged in interferences absence (0:1 ratio) and presence (0.5:1, 1:1 and 2:1 ratios), displaying so the good specificity of the proposed AuNPs sensor regarded to the global amino acids estimation on complex matrix, such as those ones of nutritional supplements.

### Example 2.5. Semi-quantitative estimation by colorimetric sensing

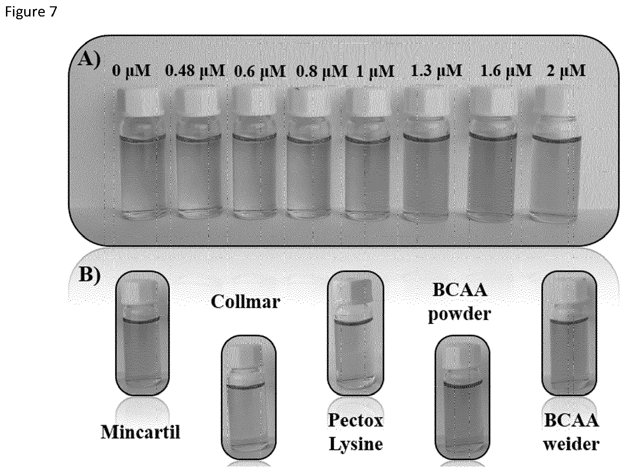

A colour guide (red-purple-blue) was developed from known standards (**Figure 7A**) with concentrations corresponding to same levels of calibration range (n=7) and where the colour changes were directly related to global amino acids amount. Based on this, a preliminary semi-quantitative estimation of total amino acids amount in tested samples can be performed by just a simple comparison between the colour guide and the different samples through the naked eye.

### Example 2.6. Analysis of Commercial Nutritional Supplements

The usefulness of the proposed sensing methodology for global amino acids amount analysis was proved by its application to different commercial samples (4 nutritional supplements and 1 pharmaceutical formulation) upon the pre-treatment already described in *section 1.3*. Solutions of 1.6 $\mu$M global amino acids concentration (upon declared amounts), from four commercial products (Mincartil, Collmar, BCCA podwer, and Pectox Lysine) and 1 $\mu$M for BCCA Weider, were prepared in the physic-chemical conditions previously selected, to get a concentration within the linear range of sensor and then their UV-Vis spectra were recorded to obtain quantitative estimations about their total amino acids amount. The results revealed as global amino acids mass (g) for each kg of sample with their respective RSD (n=5), as well as the values declared by the manufacturer, are shown in **Table 3**.

**Table 3**

| Samples | Declared by manufacturer (g/kg) | AuNPs plasmonic sensor (g/kg), (n=5) | Kjeldhal method (g/kg), (n=5) | Student's t-test* $t_0$ |
|---|---|---|---|---|
| **Mincartil tablets** | 540 | 516 $\pm$ 1.2% | 539 $\pm$ 4.0% | 2.2 |

(continued)

| Samples | Declared by manufacturer (g/kg) | AuNPs plasmonic sensor (g/kg), (n=5) | Kjeldhal method (g/kg), (n=5) | Student's t-test* $t_0$ |
|---|---|---|---|---|
| Collmar | 950 | 857 $\pm$ 2.9% | 900 $\pm$ 1.5% | 2.5 |
| Pletox Lisine | 683 | 639 $\pm$ 0.6% | 646 $\pm$ 2.0% | 1.0 |
| BCAA powder red berries | 680 | 627 $\pm$ 2.6% | 634 $\pm$ 1.2% | 0.8 |
| BCAA Weider amino acids | 619 | 971 $\pm$ 4.0% | 951 $\pm$ 1.6% | 0.8 |
| *Analysis of global amino acids amount in commercial products by the AuNPs spectrophotometric sensing and Kjeldhal methodologies, manufacturer declared values and statistical comparison between the Kjeldahl results and the quantitative sensing ones ($\alpha$ = 0.05; n = 5). * t (0.025, 4) = 2.78.* | | | | |

Very similar values to declared amounts were obtained for Mincartil, BCCA podwer, Pectox lysine and a little lower for Collmar, likely due to the presence of some small peptides instead of just free amino acids in its composition upon manufacturer indications. However, manufacturer declared value of BCCA weider seems to be underestimated according to results obtained by our plasmonic sensor and the Kjeldahl methods. Anyway, small relative standard deviations (RSD $\leq$ 4%) were obtained in all cases. Attending to this, the proposed AuNPs-based quantitative sensing methodology presents a great potential for its application to global amino acids determination in assorted and complex matrix like food supplements or pharmaceutical samples.

Moreover, it is also possible to perform preliminary semi-quantitative estimations just upon colorimetric comparisons of treated samples versus the colour guide already established in **Example 2.5** (**Figures 7A** and **7B**). As it is shown at **Figure 7B**, all the AuNPs solutions turned to intense blue when the appropriate amount of commercial sample was added. Attending to the colour guide, global amino acids concentration estimated by semi-quantitative sensor may be found in the range from 1.3 and 2 $\mu$M. Results obtained are in very good agreement with those expected according to the manufacturer specifications, except for BCCA weider product, that displays higher concentration than that specified one on its label.

As a further validation study and with the aim to prove the reliability of the proposed AuNPs sensor, the five commercial samples were also submitted to the reference official Kjeldahl method following the procedure described in bibliography by fivefold [W. Horwitz, Official methods of analysis. Association of Official Analytical Chemists Washington, DC, 1975*], to estimate total N amount, values later transformed to global amino acids amount expressed as global amino acids mass (g) for each kg of sample (**Table 3**).

Subsequently, the paired Student test-t was performed between these two sets of data (Kjeldahl and spectrophotometric ones), in order to verify the statistical accordance between results reported by both methodologies. Null and alternative hypothesis were established as $H_0$: $\mu_d$ = 0 and $H_1$: $\mu_d \neq$ 0; then to and t $_{(\alpha/2; n-1)}$ were calculated and shown in **Table 3**. Attending to the results obtained, in all cases to experimental values were lower than the theoretical one (t $_{(\alpha/2; n-1)}$); so alternative hypothesis can be rejected and therefore it can be guaranteed the absence of statistically significant differences between the results obtained by both methodologies at a confidence level of 95%.

Otherwise it should be highlighted that the sensitivity achieved by our sensing methodology is much higher (three magnitude orders) than the Kjeldahl's one (upon suggestions from the official method [W. Horwitz, Official methods of analysis. Association of Official Analytical Chemists Washington, DC, 1975], working samples solutions should be prepared in the mM order to get reliable results attending to expected sensitivity). Beside any of the two proposed methodologies (both colorimetric and spectrophotometric) will lead a quicker, simpler and less polluting alternative to that multiple, tedious and warning stages Kjeldahl method.

**Claims**

1. *In vitro* method for the estimation of global amino acids and/or small peptides content present in a sample which comprises:

   a. Contacting the sample with a solution of citrate-functionalized gold nanoparticles, wherein the concentration of the gold nanoparticles is between 0.2 and 1.6 nM, the pH of the solution is between 2.4 and 2.8 and the average diameter size of the nanoparticles is between 13 and 25 nm, and

b. Performing a quantitative estimation by measuring the absorbance ratio of the mixture between bands $A_{630-670}/A_{518}$, or a semi-quantitative estimation by comparing the intensity of developed colour versus a colour guide established from known standards.

2. The method, according to claim 1, wherein the gold nanoparticles have an average diameter size of 18 nm.

3. The method, according to any of the previous claims, wherein the concentration of the gold nanoparticles is 0.2 nM.

4. The method, according to any of the previous claims, wherein the pH of the solution is 2.6.

5. The method, according to any of the previous claims, wherein the absorbance ratio of step b) is between $A_{650}/A_{518}$.

6. The method, according to any of the previous claims, wherein the time of contacting the sample with the gold nanoparticles of step a) is up to 30 minutes, preferably 3 minutes.

7. The method, according to any of the previous claims, wherein the sample is selected from a nutritional supplement, a biological sample obtained from a subject, food samples or beverage samples.

8. The method, according to any of the previous claims, wherein the sample is a blood sample.

9. Use of a solution of citrate-functionalized gold nanoparticles, wherein the concentration of the gold nanoparticles is between 0.2 and 1.6 nM, the pH of the solution is between 2.4 and 2.8, and the average diameter size of the nanoparticles is between 13 and 25 nm, for the estimation of global amino acids and/or small peptides content present in a sample.

10. Use of a solution of citrate-functionalized gold nanoparticles, according to claim 9, for the quantitative estimation of global amino acids and/or small peptides content present in a sample, wherein the absorbance ratio of the mixture is measured between bands $A_{630-670}/A_{518}$.

11. Use of a solution of citrate-functionalized gold nanoparticles, according to claim 10, wherein the absorbance ratio is between $A_{650}/A_{518}$.

12. Use of a solution of citrate-functionalized gold nanoparticles, according to claim 9, for the semi-quantitative estimation of global amino acids and/or small peptides content present in a sample, wherein the developed colour is compared with a colour guide established from known standards.

13. Use of a solution of citrate-functionalized gold nanoparticles, according to any of the claims 9 to 12, wherein the gold nanoparticles have an average diameter size of 18 nm and their concentration is 0.2 nM.

14. Use of a solution of citrate-functionalized gold nanoparticles, according to any of the claims 9 to 13, wherein the pH value is 2.6.

15. Use of a solution of citrate-functionalized gold nanoparticles, according to any of the claims 9 to 14, wherein the time of contacting the sample with the gold nanoparticles of step a) is up to 30 minutes, preferably 3 minutes.

Figure 1

1.1

Figure 1 (Cont.)

1.2

Figure 1 (Cont.)

Figure 1 (Cont.)

Figure 1 (Cont.)

Figure 1 (Cont.)

1.3

1.4

A)

Figure 1 (Cont.)

B)

C)

D)

Figure 1 (Cont.)

E)

Figure 2

2.1

Figure 2 (Cont.)

Figure 2 (Cont.)

2.2

Figure 3

3.1

3.2

Figure 3 (Cont.)

3.3

3.4

Figure 4

Figure 5

Figure 6

Figure 7

Europäisches
Patentamt

European
Patent Office

Office européen
des brevets

# EUROPEAN SEARCH REPORT

Application Number

EP 20 38 2847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NIETZOLD C. ET AL: "Fast protein detection using absorption properties of gold nanoparticles", ANALYST, [Online] vol. 137, no. 12, 1 January 2012 (2012-01-01), page 2821, XP055776792, UK ISSN: 0003-2654, DOI: 10.1039/c2an35054h [retrieved on 2021-02-19] * the whole document * * abstract * * figures 1-7 * | 1-15 | INV. G01N33/531 G01N33/543 |
| Y | ALDEWACHI HASAN ET AL: "Study of the Stability of Functionalized Gold Nanoparticles for the Colorimetric Detection of Dipeptidyl Peptidase IV", APPLIED SCIENCES, vol. 8, no. 12, 12 December 2018 (2018-12-12), page 2589, XP055776203, DOI: 10.3390/app8122589 * the whole document * * abstract; figures 3, 5 * * figures 1,2,4 * | 1-15 | |
| Y | NASTRI FLAVIA ET AL: "Vincenzo Pavone: Friend, mentor and inspiring scientist", BIOPOLYMERS, [Online] vol. 109, no. 10, 1 October 2018 (2018-10-01), page e23234, XP055776588, US ISSN: 0006-3525, DOI: 10.1002/bip.23234 [retrieved on 2021-02-19] * the whole document * | 1-15 | |

TECHNICAL FIELDS
SEARCHED (IPC)

G01N

-/--

The present search report has been drawn up for all claims

| Place of search | Date of completion of the search | Examiner |
|---|---|---|
| The Hague | 19 February 2021 | Boiangiu, Clara |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 3

Europäisches
Patentamt

European
Patent Office

Office européen
des brevets

## EUROPEAN SEARCH REPORT

Application Number

EP 20 38 2847

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RETOUT MAURICE ET AL: "Rapid and Selective Detection of Proteins by Dual Trapping Using Gold Nanoparticles Functionalized with Peptide Aptamers", ACS SENSORS, [Online] vol. 1, no. 7, 29 June 2016 (2016-06-29), pages 929-933, XP055776525, ISSN: 2379-3694, DOI: 10.1021/acssensors.6b00229 [retrieved on 2021-02-19] * the whole document * | 1-15 | |
| Y | DAVID A. GILJOHANN ET AL: "Gold Nanoparticles for Biology and Medicine", ANGEWANDTE CHEMIE INTERNATIONAL EDITION, vol. 49, no. 19, 26 April 2010 (2010-04-26), pages 3280-3294, XP055141717, ISSN: 1433-7851, DOI: 10.1002/anie.200904359 * the whole document * | 1-15 | |
| Y | US 10 677 805 B1 (CHEN JUNG-CHIH [TW] ET AL) 9 June 2020 (2020-06-09) * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 19 February 2021 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 38 2847

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PARK JONG-WON ET AL: "Structural Study of Citrate Layers on Gold Nanoparticles: Role of Intermolecular Interactions in Stabilizing Nanoparticles", JOURNAL OF THE AMERICAN CHEMICAL SOCIETY, [Online] vol. 136, no. 5, 14 January 2014 (2014-01-14), pages 1907-1921, XP055776170, US ISSN: 0002-7863, DOI: 10.1021/ja4097384 [retrieved on 2021-02-19] * the whole document * ----- | 1-15 | |
| Y | WO 2018/220644 A1 (ADHIKARY RISHI [IN]) 6 December 2018 (2018-12-06) * the whole document * ----- | 1-15 | |
| Y | MOCANU A ET AL: "Self-assembly characteristics of gold nanoparticles in the presence of cysteine", COLLOIDS AND SURFACES A: PHYSIOCHEMICAL AND ENGINEERING ASPECTS, ELSEVIER, AMSTERDAM, NL, vol. 338, no. 1-3, 15 April 2009 (2009-04-15), pages 93-101, XP025959066, ISSN: 0927-7757, DOI: 10.1016/J.COLSURFA.2008.12.041 [retrieved on 2009-01-09] * the whole document * ----- | 1-15 | TECHNICAL FIELDS SEARCHED (IPC) |

The present search report has been drawn up for all claims

| Place of search | Date of completion of the search | Examiner |
|---|---|---|
| The Hague | 19 February 2021 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 38 2847

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2021

| Patent document cited in search report | | Publication date | Patent family member(s) | Publication date |
|---|---|---|---|---|
| US 10677805 | B1 | 09-06-2020 | NONE | |
| WO 2018220644 | A1 | 06-12-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **N. G. BASTÚS ; J. COMENGE ; V. PUNTES.** Kinetically controlled seeded growth synthesis of citrate-stabilized gold nanoparticles of up to 200 nm: size focusing versus Ostwald ripening. *Langmuir,* 2011, vol. 27 (17), 11098-11105
- **I. TIUNOV et al.** Synthesis of large uniform gold and core-shell gold-silver nanoparticles: Effect of temperature control. *Russian Journal of Physical Chemistry A,* 2016, vol. 90 (1), 152-157

- **V. BARWICK et al.** *The fitness for purpose of analytical methods,* 2014, https://www.eurachem.org/index.php/publications/guides/mv
- **T. WENZL ; J. HAEDRICH ; A. SCHAECHTELE ; P. ROBOUCH ; J. STROKA.** *Guidance Document on the Estimation of LOD and LOQ for Measurements in the Field of Contaminants in Feed and Food,* 2016
- **W. HORWITZ.** *Official methods of analysis. Association of Official Analytical Chemists Washington,* 1975